# EUROPÄISCHE PATENTSCHRIFT

(11) **EP 2 822 512 B1**
(45) Veröffentlichungstag und Bekanntmachung des Hinweises auf die Patenterteilung: **11.10.2017**
(21) Anmeldenummer: 13709386.0
(22) Anmeldetag: 06.03.2013
(51) Int. Cl.: A61F 2/46, A61F 2/34, A61F 2/30

(54) **VORRICHTUNG ZUR PRÜFUNG EINES KERAMISCHEN PFANNENEINSATZES FÜR HÜFTGELENKSIMPLANTATE**
DEVICE FOR TESTING A CERAMIC SOCKET INSERT FOR HIP JOINT IMPLANTS
DISPOSITIF DE CONTRÔLE D'UN INSERT DE CAVITÉ CÉRAMIQUE POUR DES IMPLANTS D'ARTICULATION COXO-FÉMORALE

(30) Priorität: 06.03.2012 DE 102012203513
(43) Veröffentlichungstag der Anmeldung: 14.01.2015
(73) Patentinhaber: CeramTec GmbH, 73207 Plochingen (DE)
(72) Erfinder: FLOHR, Markus, 73733 Esslingen (DE); BERTMARING, Hendrik, 73732 Esslingen (DE); HÄUSSLER, Kim,Lars, 73728 Esslingen (DE); GRAF, Helena, 70188 Stuttgart (DE)
(74) Vertreter: Uppena, Franz
(86) Internationale Anmeldenummer: PCT/EP2013/054469
(87) Internationale Veröffentlichungsnummer: WO 2013/131938

(56) Entgegenhaltungen:
- EP-A1- 0 921 771
- DE-A1- 19 841 826
- DE-B1- 2 418 956

## Beschreibung

Die Erfindung betrifft eine Vorrichtung zur Prüfung eines keramischen Pfanneneinsatzes nach dem Oberbegriff des Anspruchs 1.

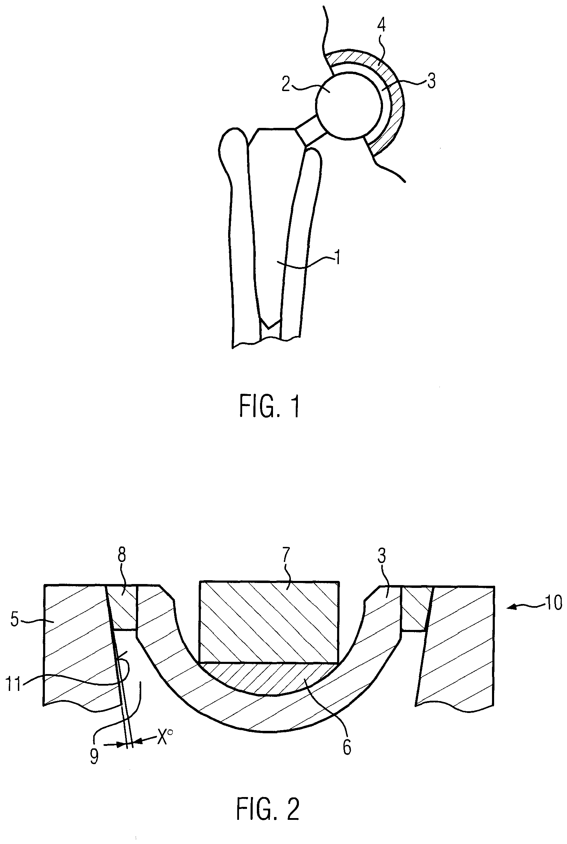

Im Markt existiert eine Vielzahl von Prothesen-Systemen zum Ersatz des natürlichen Hüftgelenkes. Diese bestehen, wie in Figur 1 dargestellt, in der Regel aus einem Schaft 1 gekoppelt mit einem Kugelkopf 2 und einer künstlichen Hüftpfanne 4 gekoppelt mit einem Pfanneneinsatz 3. Schaft 1 und Hüftpfanne 4 sind mit dem Körper meist durch Einwachsen in den Oberschenkel- bzw. Beckenknochen verbunden und sind Träger für den Kugelkopf 2 bzw. Pfanneneinsatz 3. Der Kugelkopf 2 ist in der Kalotte des Pfanneneinsatzes 3 drehbar gelagert und bildet mit diesem eine Gleitpaarung. Als Gleitpartner kommen verschiedene Materialien, u.a. Keramik, zum Einsatz. Auf der acetabulären Seite der Gleitpaarung existieren wie oben beschrieben modulare Systeme, das heißt Hüftpfanne und Pfanneneinsatz werden separat geliefert und dementsprechend erst *in situ* gefügt. Daneben existieren auch monolithische Systeme, die nur eine einzige acetabuläre Komponente aufweisen und direkt in den Beckenknochen eingesetzt werden, und vorgefügte Systeme, bei denen schon werksseitig Hüftpfanne und Pfanneneinsatz miteinander verbunden werden.

Die erforderliche Mindestfestigkeit von keramischen acetabulären Hüftgelenksimplantaten wird häufig über einen Prooftest (100%-Prüfung) sichergestellt. Dabei wird das acetabuläre Hüftgelenksimplantat mechanisch belastet. Beschrieben ist dies in EP 0 921 771 A1 oder DE 198 41 826; die letztgenannte Druckschrift zeigt die Merkmale des Oberbegriffs von Anspruch 1. 100%-Prüfung bedeutet, dass die Bauteile vor der Auslieferung mechanisch belastet werden und Bauteile mit kritischen Fehlern im Prooftest zerbrechen, so dass nur Bauteile mit ausreichend hoher mechanischer Festigkeit den Prooftest überstehen.

Bisher werden verschiedene Testaufbauten für die vielen spezifischen Geometrien der acetabulären Hüftgelenksimplantate angewendet. Dies erfordert eine Vielzahl an Testaufbauten und damit auch eine große Lagerkapazität.

Der Erfindung liegt die Aufgabe zugrunde, eine Vorrichtung nach dem Oberbegriff des Anspruchs 1 so zu verbessern, dass sie universell für alle acetabulären Hüftgelenksimplantate (monolithisch, modular, vorgefügt) eingesetzt werden kann und somit alle derzeitigen Vorrichtungen ersetzt.

Erfindungsgemäß wird diese Aufgabe durch die Merkmale des Anspruchs 1 gelöst.

Dadurch, dass im Positionierbereich zwischen der Aufnahmevorrichtung und dem Pfanneneinsatz ein ringförmiges duktiles Adaperstück mit einer konischen Außenfläche, die am Aufnahmekonus und einer Innenfläche, die am Pfanneneinsatz anliegt, angeordnet ist, wobei die Reibung zwischen Aufnahmevorrichtung und Adapterstück niedriger ist als zwischen Adapterstück und Pfanneneinsatz, wird unter Last eine Relativbewegung hauptsächlich zwischen Aufnahmevorrichtung und Adapterstück erzeugt. Dies gewährleistet in Kombination mit der konisch gestalteten Aufnahmevorrichtung bzw. dem Aufnahmekonus eine Erhöhung der radial wirkenden Kraftkomponente. Hierdurch kann die erfindungsgemäße Vorrichtung universell für alle acetabulären Hüftgelenksimplantate (monolithisch, modular, vorgefügt) eingesetzt werden.

Durch einen anzustrebenden konstanten Spannungsverlauf an der Außenkontur des Pfanneneinsatzes, werden nahezu alle Lastsituationen, die der Pfanneneinsatz bzw. das Implantat unter in vivo Bedingungen erfahren kann, abgedeckt.

Bevorzugt ist der Aufnahmekonus zumindest im Positionierbereich poliert. Hierdurch gleitet das Adapterstück leichter auf dem Aufnahmekonus bzw. der Aufnahmevorrichtung.

In einer Ausgestaltung der Erfindung besteht das Adapterstück aus Messing und die Aufnahmevorrichtung aus oberflächengehärtetem Stahl.

Bevorzugt ist zwischen der konischen Außenfläche des Adaperstücks und dem Aufnahmekonus und/oder zwischen der Innenfläche des Adapterstücks und dem Pfanneneinsatz ein Winkelspalt x° angeordnet, wobei sich der Winkelspalt x° ausgehend vom oberen, zum Pfanneneinsatz gewandten Ende des Aufnahmekonus nach unten vergrößert.

Dadurch ist gewährleistet, dass sich die Aufnahmefläche bzw. Kontaktfläche unter zunehmender Last zwischen Aufnahmevorrichtung und Adapterstück und/oder zwischen Adapterstück und Pfanneneinsatz von der Stirnseite des Pfanneneinsatzes ausgehend ausbildet. Der Winkelspalt x° bzw. die Winkelspalte stellen sicher, dass der Pfanneneinsatz entsprechend der späteren *in vivo* Anwendung belastet wird. Zusätzlich werden Zugspannungen bereits im Konusbereich erzeugt.

Der Winkelspalt stellt sicher, dass sich die Zugspannungen von der Stirnfläche aus, über den Konusbereich in Richtung Rückseite ausbilden/fortsetzen. Dies kann für unterschiedliche Bauteile unterschiedlich ausfallen. Bevorzugt liegt der Winkelspalt x° zwischen 30 Winkelsekunden und 20 Winkelminuten. Ganz besonders bevorzugt zwischen 6 und 10 Winkelminuten. In einer Ausführungsform haben sich besonders 8 Winkelminuten als vorteilhaft herausgestellt. Mit zunehmender Belastung soll dieser Winkelspalt überbrückt werden und wird deshalb nach oben begrenzt. Die Toleranzgrenzen hängen unter Umständen von der Belastunghöhe ab.

In einer Ausführungsform mit einem Stempel zur Kraftbeaufschlagung des Druckstücks ist das Material des Stempels härter als das Material des Druckstücks, wobei bevorzugt der Stempel aus gehärtetem Stahl und das Druckstück aus einem Kunststoff besteht. Teflon ist vorzüglich geeignet.

Die Pfanneneinsätze, auch Implantat genannt, werden also beim Prooftest über eine Aufnahmevorrichtung positioniert und anschließend belastet. Zwischen Implantat und Aufnahmevorrichtung befindet sich eine Adapterstück aus duktilem Material. Dadurch ist sicher gestellt, dass sich das Adapterstück an das Implantat anpassen kann. Idealerweise besteht das Adapterstück aus Messing und die Aufnahmevorrichtung aus oberflächengehärtetem Stahl. Der Bereich der Aufnahmevorrichtung, in dem das Adapterstück und das Implantat positioniert werden, ist konisch gestaltet. Dadurch werden eingeleitete Kräfte in ihre normal und radial wirkenden Komponenten zerlegt. Das Material der Aufnahmevorrichtung ist im Positionierbereich so bearbeitet, dass die Reibung zwischen Aufnahmevorrichtung und Adapterstück niedriger ist als zwischen Adapterstück und Implantat. Im Idealfall sollte dieser Bereich poliert sein. Dadurch wird unter Last eine Relativbewegung hauptsächlich zwischen Aufnahmevorrichtung und Adapter erzeugt. Dies gewährleistet in Kombination mit der konisch gestalteten Aufnahmevorrichtung eine Erhöhung der radial wirkenden Kraftkomponente.

Die eigentliche Lasteinleitung kann sowohl über die Stirnfläche oder Kalotte des Implantats, als auch über das Adapterstück erfolgen. Idealerweise wird die Last aber über einen Stempel und eines daran befestigten Druckstücks, das an die Innenkontur des Implantats angepasst ist, eingeleitet. Das Material des Druckstücks, das an den Stempel adaptiert wird, sollte ausreichend duktil sein, um unter Last eine Anpassung an die exakte Innenkontur des Implantats zu gewährleisten. Idealerweise besteht das Druckstück aus Teflon. Der Stempel muss härter sein als das Druckstück und sollte vorzugsweise aus gehärtetem Stahl bestehen. Somit wird auf der Außenkontur ein möglichst konstanter Spannungsverlauf erzeugt.
Figur 1 zeigt ein bekanntes Hüftgelenksimplantat. Beschrieben ist es in der Beschreibungseinleitung.
Figur 2 zeigt eine erfindungsgemäße Vorrichtung zur Prüfung eines keramischen Pfanneneinsatzes 3 eines Hüftgelenksimplantats. Die Vorrichtung besteht aus einer Aufnahmevorrichtung 5, einem Druckstück 6 und einem Stempel 7, wobei in der Aufnahmevorrichtung 5 eine Ausnehmung 9 mit einem Positionierbereich 10 zur Aufnahme des Pfanneneinsatzes (3) angeordnet ist und die Ausnehmung 9 im Positionierbereich 10 einen Aufnahmekonus 11 aufweist.

Im Positionierbereich 10 zwischen der Aufnahmevorrichtung 5 und dem Pfanneneinsatz 3 ist ein ringförmiges duktiles Adaperstück 8 aus Messing mit einer konischen Außenfläche, die am Aufnahmekonus 11 und einer Innenfläche, die am Pfanneneinsatz anliegt, angeordnet. Die Reibung zwischen Aufnahmevorrichtung 5 und Adapterstück 8 ist niedriger als zwischen Adapterstück 8 und Pfanneneinsatz 3.

In einer alternativen Ausführungsform, hier nicht gezeigt, kann der Winkelspalt auch oder nur zwischen der Innenfläche des Adapterstücks und dem Pfanneneinsatz angeordnet sein.

Bei der Prüfung eines Pfanneneinsatzes 3 wird dieser über ein ringförmiges Adapterstück 8 in die Aufnahmevorrichtung 5 eingesetzt. Anschließend wird über den Stempel 7 das Druckstück 6 belastet. Sollte der Pfanneneinsatz 3 brechen, war er fehlerhaft. Bricht er nicht, so ist sichergestellt, dass er bei Kräften unterhalb der aufgebrachten Kraft nicht bricht.

Wesentlich an der Vorrichtung ist, dass die Reibung zwischen Aufnahmevorrichtung und Adapterstück niedriger ist als zwischen Adapterstück und Pfanneneinsatz. Denn dadurch wird unter Last eine Relativbewegung hauptsächlich zwischen Aufnahmevorrichtung und Adapterstück erzeugt. Dadurch wird ein möglichst konstanter Spannungsverlauf an der Außenkontur des Pfanneneinsatzes erzeugt, so dass diese Vorrichtung für alle Pfanneneinsätze verwendet werden kann.

## Patentansprüche

1. Vorrichtung zur Prüfung eines keramischen Pfanneneinsatzes (3) von Hüftgelenksimplantaten mit einer Aufnahmevorrichtung (5), einem Druckstück (6), einem Pfanneneinsatz (3) und optional einem Stempel (7), wobei die Aufnahmevorrichtung (5) eine Ausnehmung (9) mit einem Positionierbereich (10) zur Aufnahme des Pfanneneinsatzes (3) aufweist und die Ausnehmung (9) im Positionierbereich (10) einen Aufnahmekonus (11) aufweist, wobei im Positionierbereich (10) zwischen der Aufnahmevorrichtung (5) und dem Pfanneneinsatz (3) ein ringförmiges duktiles Adapterstück (8) mit einer konischen Außenfläche, die am Aufnahmekonus (11) und einer Innenfläche, die am Pfanneneinsatz anliegt, angeordnet ist, **dadurch gekennzeichnet, dass** die Reibung zwischen Aufnahmevorrichtung (5) und Adapterstück (8) niedriger ist als zwischen Adapterstück (8) und Pfanneneinsatz (3), und **dadurch gekennzeichnet, dass** zwischen der konischen Außenfläche des Adaperstücks (8) und dem Aufnahmekonus (11) der Ausnehmung (9) und/oder zwischen der Innenfläche des Adapterstücks und dem Pfanneneinsatz ein Winkelspalt x° angeordnet ist, wobei sich der Winkelspalt x° ausgehend vom oberen, zum Pfanneneinsatz (3) gewandten Ende des Aufnahmekonus (11) nach unten vergrößert.

2. Vorrichtung nach Anspruch 1, **dadurch gekennzeichnet, dass** der Aufnahmekonus (11) zumindest im Positionierbereich poliert ist.

3. Vorrichtung nach Anspruch 1 oder 2, **dadurch gekennzeichnet, dass** das Adapterstück (8) aus Messing besteht.

4. Vorrichtung nach einem der Ansprüche 1 bis 3, **dadurch gekennzeichnet, dass** die Aufnahmevorrichtung (5) aus oberflächengehärtetem Stahl besteht.

5. Vorrichtung nach einem der Ansprüche 1 bis 4, **dadurch gekennzeichnet, dass** der Winkelspalt x° zwischen 30 Winkelsekunden und 20 Winkelminuten, bevorzugt zwischen 6 und 10 Winkelminuten liegt.

6. Vorrichtung nach Anspruch 5, **dadurch gekennzeichnet, dass** der Winkelspalt x° 8 Winkelminuten beträgt.

7. Vorrichtung nach einem der Ansprüche 1 bis 6 mit einem Stempel (7) zur Kraftbeaufschlagung des Druckstücks (6), **dadurch gekennzeichnet, dass** das Material des Stempels (7) härter ist als das Material des Druckstücks (6), wobei bevorzugt der Stempel (7) aus gehärtetem Stahl und das Druckstück (6) aus Kunststoff, bevorzugt Teflon besteht.

## Claims

1. A device for testing a ceramic socket insert (3) of hip joint implants with a receiving device (5), a pressure piece (6), a socket insert (3) and optionally a punch (7), wherein the receiving device (5) has a recess (9) with a positioning region (10) for receiving the socket insert (3), and the recess (9) has in the positioning region (10) a receiving cone (11), wherein arranged in the positioning region (10) between the receiving device (5) and the socket insert (3) there is an annular ductile adapter piece (8) with a conical outer surface that rests against the receiving cone (11) and an inner surface that rests against the socket insert, **characterised in that** the friction between the receiving device (5) and the adapter piece (8) is lower than between the adapter piece (8) and the socket insert (3), and **characterised in that** arranged between the conical outer surface of the adapter piece (8) and the receiving cone (11) of the recess (9) and/or between the inner surface of the adapter piece and the socket insert there is an angular gap x°, wherein the angular gap x° increases downwards starting from the upper end of the receiving cone (11) facing the socket insert (3).

2. A device according to claim 1, **characterised in that** the receiving cone (11) is polished at least in the positioning region.

3. A device according to claim 1 or 2, **characterised in that** the adapter piece (8) consists of brass.

4. A device according to one of claims 1 to 3, **characterised in that** the receiving device (5) consists of surface-hardened steel.

5. A device according to one of claims 1 to 4, **characterised in that** the angular gap x° lies between 30 angular seconds and 20 angular minutes, preferably between 6 and 10 angular minutes.

6. A device according to claim 5, **characterised in that** the angular gap x° amounts to 8 angular minutes.

7. A device according to one of claims 1 to 6 having a punch (7) to apply the force of the pressure piece (6), **characterised in that** the material of the punch (7) is harder than the material of the pressure piece (6), wherein preferably the punch (7) consists of hardened steel, and the pressure piece (6) consists of plastics material, preferably Teflon.

## Revendications

1. Dispositif de contrôle d'un insert de cavité (3) céramique pour implants d'articulation coxofémorale, comprenant un dispositif de réception (5), un élément de pression (6), un insert de cavité (3) et, de manière facultative, un poussoir (7), sachant que le dispositif de réception (5) présente un évidement (9) doté d'une zone de positionnement (10) destinée à recevoir l'insert de cavité (3) et que l'évidement (9) présente un cône de réception (11) dans la zone de positionnement (10), sachant que dans la zone de positionnement (10), un élément adaptateur (8) annulaire ductile est placé entre le dispositif de réception (5) et l'insert de cavité (3), lequel élément est doté d'une surface externe conique, appliquée contre le cône de réception (11), et d'une surface interne appliquée contre l'insert de cavité, **caractérisé en ce que** le frottement entre le dispositif de réception (5) et l'élément adaptateur (8) est plus faible que celui entre l'élément adaptateur (8) et l'insert de cavité (3), et **caractérisé en ce qu'**un espace angulaire x° est prévu entre la surface externe conique de l'élément adaptateur (8) et le cône de réception (11) de l'évidement (9) et/ou entre la surface interne de l'élément adaptateur et l'insert de cavité, ledit espace angulaire x° allant en s'élargissant vers le bas, à partir de l'extrémité supérieure du cône de réception (11) tournée vers l'insert de cavité (3).

2. Dispositif selon la revendication 1, **caractérisé en ce que** le cône de réception (11) est poli au moins dans la zone de positionnement.

3. Dispositif selon la revendication 1 ou 2, **caractérisé en ce que** l'élément adaptateur (8) est en laiton.

4. Dispositif selon l'une des revendications 1 à 3, **caractérisé en ce que** le dispositif de réception (5) est en acier trempé superficiellement.

5. Dispositif selon l'une des revendications 1 à 4, **caractérisé en ce que** l'espace angulaire x° est compris entre 30 secondes d'arc et 20 minutes d'arc, de préférence entre 6 et 10 minutes d'arc.

6. Dispositif selon la revendication 5, **caractérisé en ce que** l'espace angulaire x° est de 8 minutes d'arc.

7. Dispositif selon l'une des revendications 1 à 6, comprenant un poussoir (7) pour l'application d'une force à l'élément de pression (6),**caractérisé en ce que** le matériau du poussoir (7) est plus dur que le matériau de l'élément de pression (6), le poussoir (7) étant de préférence constitué d'acier trempé et l'élément de pression (6) de matière plastique, de préférence de téflon.
